# EUROPÄISCHE PATENTSCHRIFT

(11) **EP 1 894 590 B1**
(45) Veröffentlichungstag und Bekanntmachung des Hinweises auf die Patenterteilung: **25.04.2012**
(21) Anmeldenummer: 06119779.4
(22) Anmeldetag: 30.08.2006
(51) Int. Cl.: A61M 5/315

(54) **Injektionsvorrichtung mit vereinfachter Stopfenrückhaltung**
Injection device with simplified retaining of a stopper
Dispositif d'injection avec simplification de la retenue d'un bouchon

(43) Veröffentlichungstag der Anmeldung: 05.03.2008
(73) Patentinhaber: Roche Diagnostics GmbH, 68305 Mannheim (DE); F.Hoffmann-La Roche AG, 4070 Basel (CH)
(72) Erfinder: Heutschi, Ivan, 3506, Grosshöchstetten (CH)
(74) Vertreter: Rentsch Partner AG

(56) Entgegenhaltungen:
- US-A1- 2002 016 572
- US-A1- 2005 043 689
- US-A1- 2006 173 409

## Beschreibung

Die Erfindung betrifft eine Injektionsvorrichtung mit einem Volumen zur Aufnahme eines zu verabreichenden Produkts, mit einem Kolben, um auf das Produkt in dem Volumen einzuwirken, und mit einem Stopfen, der zwischen dem produktseitigen Ende des Kolbens und dem im Volumen befindlichen Produkt anordenbar ist, gemäß dem Anspruch 1.

Im Stand der Technik werden Infusionsvorrichtungen eingesetzt, die eine Rückhalterung für den eingesetzten Stopfen über ein Gewinde oder ein Überdruckventil zum Einsatz bringen. Bei derartigen Injektionsvorrichtungen treten somit relativ hohe Kosten auf und die Handhabung ist umständlich und folglich risikobehaftet. Es ist eine Zielsetzung der Erfindung, wenigstens einen Nachteil nach dem Stand der Technik wenigstens teilweise auszuräumen. Insbesondere sollen eine Injektionsvorrichtung mit einer Rückhalterung für den eingesetzten Stopfen zur Verfügung gestellt werden, die relativ einfach aufgebaut ist und die folglich kostengünstiger ist als Vorrichtungen der bekannten Art.

Ein Injektionsvorrichtung gemäss dem Oberbegriff von Anspruch 1 ist aus dem Dokument US 2006/173409 A1 bekannt.

Ferner kann es als eine Zielsetzung gemäß der Erfindung angesehen werden, eine einfach handhabbare Injektionsvorrichtung zur Verfügung zu stellen.

Gemäß der Erfindung beruhen deren Vorteile darauf, dass der Kolben mit einem gasdicht abdichtbaren Kolbenteller ausgestattet ist, der separat zu dem Stopfen angeordnet ist, so dass ein Unterdruck- oder Vakuumbereich zwischen dem Kolbenteller und dem Stopfen ausbildbar ist.

Bei der erfindungsgemäßen Injektionsvorrichtung wird folglich die Stopfenrückhaltung durch die Erzeugung eines Unterdrucks oder eines Vakuums verwirklicht. Während der Stopfen in der üblichen Weise durch den Kolbenteller vorwärts bewegt wird, lässt der Unterdruckbereich bzw. Vakuumbereich zwischen dem Stopfen und dem Kolbenteller eine ungewollte Bewegung des Stopfens nicht zu, da der Unterdruck im Zwischenraum durch eine Bewegung des Stopfens ohne Mitbewegung des Tellers verstärkt werden würde, so dass eine ungewollte, separate Bewegung des Stopfens entweder wesentlich verringert oder gedämpft wird, oder aber, wenn kleinere bzw. keine zu großen Kräfte auf den Stopfen einwirken, werden derartige ungewollte Bewegungen von vorne herein verhindert.

Ist ein Patient über eine herkömmliche Injektionsvorrichtung und ein zugehöriges Infusionsset zu einer Versorgung angeschlossen, so können auf den Stopfen verschieden Kräfte einwirken. Zu diesen potentiellen Kräften gehören ein Unterdruck in der Umgebung relativ zu dem Druck in dem Volumen der Injektionsvorrichtung und der hydrostatische Druck hervorgerufen durch die Höhendifferenz der Einstichstelle des Infusionssets am Körper des Patienten und der Injektionsvorrichtung.

Zu berücksichtigen ist bei der Erfindung wie auch beim Stand der Technik, dass zusätzliche Reibungskräfte zwischen der Volumenwandung der Injektionsvorrichtung und dem Stopfen auftreten. Diese Reibungskräfte können sehr unterschiedlich ausfallen, so dass hier wirklich reproduzierbare Verabreichungsparameter nicht zur Verfügung stehen. D.h., während bei einer Injektionsvorrichtung der Stopfen bei relativ hohen Kräften, die durch die Umwelt auf ihn einwirken, keine ungewollte Verschiebung erfährt, kann bei einer anderen, identisch aufgebauten Injektionsvorrichtung eine ungewollte Verschiebung in der gleichen Umgebung ohne Weiteres auftreten.

Gemäß der Erfindung hat sich nun herausgestellt, dass der Unterdruck- bzw. Vakuumbereich, der gemäß der Erfindung zwischen dem Stopfen und dem Kolbenteller bereitgestellt wird, eine Bandbreite von Kräften bis zu großen Kräften zur Verfügung stellt, um den Stopfen gegen die angreifenden Kräfte abzusichern, um eine ungewollte Verabreichung eines Medikaments oder Produkts vermeiden zu können.

Eine vorteilhafte Ausführungsform gemäß der Erfindung ergibt sich, wenn der Kolbenteller eine vollumfängliche Dichtlippe aufweist, die gegenüber der Volumenwandung gasdicht abschließt. Auf diese Weise kann der Kolbenteller hin- und herbewegt werden, wobei sich zwischen dem Stopfen und dem Kolbenteller ein Vakuum bzw. ein Unterdruckbereich einrichten lässt. Andererseits ist es auch möglich, den Kolbenteller ohne Dichtlippe hinreichend passgenau auszubilden, so dass er gegenüber der Volumenwandung abzudichten vermag, um einen Unterdruck bzw. ein Vakuum zwischen dem Stopfen und dem Teller bewerkstelligen zu können.

Vorteilhafterweise kann, insofern eine Dichtlippe vorgesehen ist, diese entgegen der Einführungsrichtung des Kolbentellers eine Sollundichtigkeitseinrichtung aufweisen. Im einfachsten Falle kann die Dichtlippe beim Einschieben des Kolbentellers in das Volumen entgegen der Einschubrichtung des Kolbentellers in das Volumen umschnappen und somit eine Undichtigkeit bewirken, über die Luft bzw. Gas innerhalb des freien Volumens, d.h., bis der Kolbenteller auf den Stopfen stößt, entweichen kann. Wird anschließend, nachdem der Teller auf den Stopfen getroffen ist, an dem Kolbenteller gezogen, wird ein Unterdruckbereich eingerichtet, wobei die Dichtlippe an ihrem entsprechenden Außenumfang so ausgebildet ist, dass sie Dichtwirkung bewerkstelligen kann. Dabei kann die Dichtlippe beispielsweise auf ihrer dem Stopfen zugewandten Seite am Rand oder nahe dem Rand mit einer Struktur etwa einer radial erstreckten Rillenstruktur versehen sein, die die Dichtlippe in Axialrichtung der Dichtlippe bzw. des Kolbentellers nicht durchzieht.

Zur Erzeugung des Vakuums zwischen dem Stopfen und dem Kolbenteller muss es nicht notwendig sein, am Kolbenteller zu ziehen, d. h., es ist keine Rückwärtsbewegung des Kolbentellers notwendig, um das Vakuum zu erzeugen. Ein Umklappen der Dichtlippe kann zum Lösen des Kolbentellers vom Stopfen benötigt werden, da dadurch Luft in den Zwischenraum einströmen kann und somit das Vakuum aufgehoben bzw. die Verbindung Stopfen-Kolbenteller gelöst wird.

Andererseits ist es auch möglich, die Kolbenstange, an der der Kolbenteller festgelegt ist, so auszubilden, dass über diesen der beim Einschieben des Kolbentellers auftretende Überdruck entweichen kann. So kann beispielsweise die Kolbenstange teilweise hohl ausgebildet sein bzw. mit einer inneren Leitung ausgebildet sein, die sich öffnen und verschließen lässt, so dass ein Überdruck über die Kolbenstange entweichen kann. Nachdem der Überdruck entwichen ist, wird die Leitung verschlossen, etwa mittels eines Stopfens, eines Ventils, einer Folie oder dgl., so dass beim nachfolgenden Belasten des Stopfens bzw. des Kolbentellers ein ungewolltes Verschieben des Stopfens durch den sich aufbauenden Unterdruck verhindert werden kann.

Natürlich ist es auch möglich den Kolbenteller mit einer andersartigen Dichteinrichtung auszubilden, etwa einem separaten oder angespritzten O-Ring. Dieser O-Ring vermag dann ebenfalls gegenüber der Wandung des Volumens abzudichten.

Ferner kann ein derartiger Teller bzw. ein Teller an sich gemäß der Erfindung auch mit wenigstens einem Ventil ausgebildet sein. Ein solches Ventil kann beispielsweise in der Form eines Ein-Wege-Ventils vorgesehen werden, d.h., dieses Ventil öffnet sich wenn der Kolbenteller in das Volumen eingeschoben wird, so dass ein Überdruck entweichen kann. Nach dem Entweichen des Überdrucks oder beim Anziehen an der Kolbenstange schließt sich das Ein-Wege-Ventil und ein Vakuum kann zwischen dem Teller und dem Stopfen aufgebaut werden, das wie voranstehend erläutert, seine positiven Wirkungen entfalten kann. Das Ventil kann ein Einströmen von Luft ins Vakuum verhindern. Dadurch hat jede noch so kleine Verschiebung des Stopfens einen Unterdruck im Volumen zur Folge, welcher gegen potentiell angreifenden Kräften wie Unterdruck in der Umgebung oder hydrostatische Druckkräfte wirkt. Je größer die ungewollte Verschiebung des Stopfens vom Kolben weg ist, desto größer wird die durch den Unterdruck aufgebaute Gegenkraft, die den Kolben in die Ausgangslage zurück bewegt.

Ferner kann als Ventileinrichtung -auch ein Zwei-Wege-Ventil eingesetzt werden, insbesondere wenn dieses mit unterschiedlichen Auslösedrücken ausgebildet ist. So kann der Auslösedruck beim Einschieben des Kolbentellers niedriger eingestellt sein, als der Auslösedruck beim Herausziehen des Kolbentellers. Dabei muss beachtet werden, dass der Auslösedruck beim Herausziehen respektive die dadurch aufbringbare Rückhaltekraft, größer ist als die maximal potentiell wirkenden Umweltzugkräfte auf den Stopfen.

Auf diese Weise ist es möglich, die prinzipiell wieder verwendbare Injektionsvorrichtung mit Merkmalen gemäß der Erfindung auch hier besonders einfach wieder zu verwenden. Wird der beim Herausziehen höher eingestellte Auslösedruck des Zwei-Wege-Ventils überwunden, kann der Kolbenteller an der Kolbenstange herausgezogen werden und das Volumen kann gereinigt werden, um die gesamte Vorrichtung gemäß der Erfindung wieder zu verwenden. Andererseits ist es auch möglich, das Volumen mit dem Stopfen nach der Entleerung bzw. nach dem Gebrauch zu entsorgen und ein anderes Volumen mit frischem Produkt bzw. Medikament oder dgl. zu verwenden, und den selben Kolbenteller, eventuell nach einer Reinigungsprozedur, in einem neuen Volumen bzw. mit einer neuen Ampulle abermals einzusetzen oder aber einen neuen Kolbenteller zu verwenden.

Vorteilhafterweise kann der Stopfen mit einer Kavität ausgebildet sein, die sich in Axialrichtung des Stopfens gegenüber dem Kolbentelter befindet, um die Stülpbewegung der Dichtlippe zu begünstigen, wobei der Unterdruckbereich zwischen Teller und Stopfen ausgebildet wird.

Natürlich können auch zwei Ventile entsprechend dem voranstehend erörterten Zwei-Wege-Ventil dimensioniert und separat vorgesehen werden, anstelle eines einzelnen Zwei-Wege-Ventils mit den voranstehend aufgeführten Merkmalen.

Eine weitere kostengünstige Ausbildung einer Ventileinrichtung für die erfindungsgemäße Vorrichtung ergibt sich, wenn eine Durchlassöffnung eines Ventils mit einer Membran abgeschlossen ist, die in Luft- oder Gasauslassrichtung frei oder eingeschränkt frei bewegbar ist, während die Membran in Lufteinlassrichtung abgestützt ist. Durch die Abstützung in Lufieintassrichtung kann eine dichtende Anlage zur Membranabstützung, d.h., zur Umgebung der Ventilöffnung, erlangt werden, so dass zwischen dem Stopfen und dem Kolbenteller ein hinreichender Unterdruck- oder Vakuumbereich ausbildbar ist.

Gemäß der Erfindung hat es sich herausgestellt, dass ein kleinerer Unterdruck- bzw. Vakuumbereich zwischen dem Kolbenteller und dem Stopfen zu einer größeren Rückhaltekraft für den Stopfen führt, die gegen ein ungewolltes Verschieben des Stopfens wirkt. Nachfolgend wird eine Tabelle wiedergegeben, die einen Überblick der Haltekräfte (F) bei einer Verschiebung (dX) des Stopfens bei Stillstand des Kolbentellers zeigt.

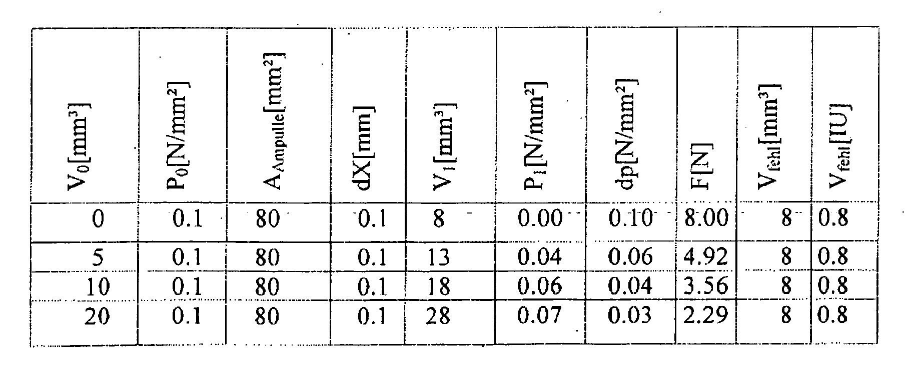

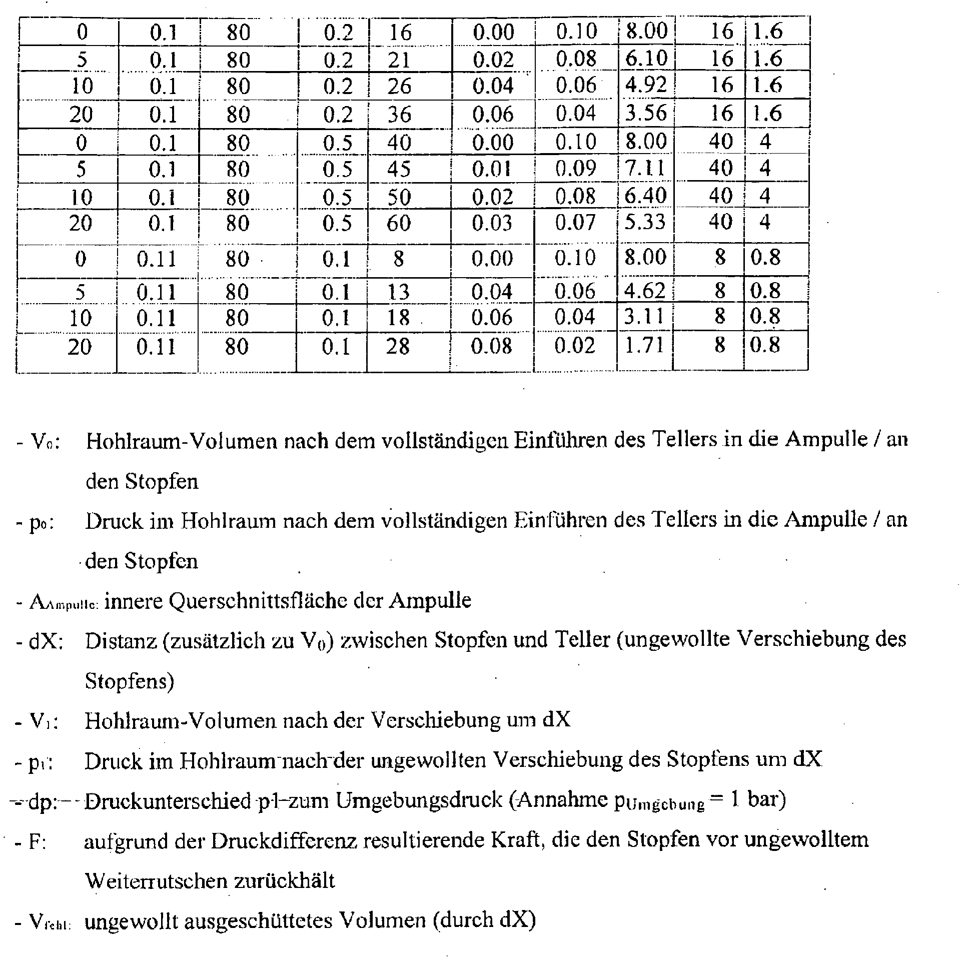

Die Haltekraft, die erfindungsgemäß durch das zwischen dem Stopfen und dem Teller aufbaubare Vakuum bzw. der entsprechende Unterdruck bewirkt werden kann, hängt dabei auch noch von Parametern ab, wie etwa dem Ampullenquerschnitt, d.h., dem Querschnitt des Volumens mit dem zu verabreichenden Produkt, und dem Volumen des Unterdruck- bzw. Vakuumbereichs vor der Verschiebung des Kolbentellers, wobei dieses Volumen als V₀ bezeichnet wird. Ferner spielt auch der Druck p₀ in dem Unterdruck- bzw. Vakuumbereich vor dem Verschieben eine Rolle. Die Luft in dem Unterdruck- bzw. Vakuumbereich muss beim Einführen des Tellers leicht entweichen können.

Wird der Stopfen bei der Vorrichtung gemäß der Erfindung um eine in der Tabelle angegebene Strecke dX verschoben, so wird dem Patienten versehentlich ein Volumen von V_{feh1} eines Medikamentes oder dgl. zugeführt. Der Stopfen kann sich hier lediglich dadurch ungewollt verschieben, dass der Druck in dem vorderen Teil des Flüssigkeitspfades zur Zufuhr des Medikaments bzw. des Produktes kleiner ist als der Druck in dem Unterdruck- bzw. Vakuumbereich. Dieser kleinere Druck im vorderen Teil des Flüssigkeitspfades kann aufgrund von Kräften auftreten, die auf das Gesamtkräftediagramm einwirken, etwa der Unterdruck in der Umgebung oder der hydrostatische Druck im gesamten Infusionsset. Ferner muss, damit sich der Stopfen nach vorne bewegt, zusätzlich noch die Stopfenreibung überwunden werden.

Die Tabelle ist unter der Prämisse erstellt worden, dass die Querschnittsfläche des Volumens mit dem Produkt ca. 80 mm² beträgt, wobei ein Unterdruck in der Umgebung der erfindungsgemäßen Vorrichtung in einem Bereich von ± 150 mb liegt, wodurch sich eine zusätzliche Kraftausübung von ±1,2 Nmax ergeben kann. Der hydrostatische Druck kann bei 1 m Flüssigkeitssäule im Infusions-Set etwa ±0,8 N betragen. Dementsprechend ist es vorteilhaft, wenn die Haltekräfte, die durch den Unterdruck- bzw. Vakuumbereich zwischen dem Kolbenteller und dem Stopfen aufgebracht werden können, die in einem Bereich variierenden ungewollten Angriffskäfte jedenfalls wenigstens etwas übersteigen, so dass eine ungewollte Verschiebung und damit eine ungewollte Verabreichung eines Medikaments nicht erfolgen kann.

Nachfolgend wird die vorliegende Erfindung anhand von bevorzugten Ausführungsformen unter Bezugnahme auf die beigefügten Figuren näher erläutert. Dabei werden weitere Vorteile, Merkmale sowie Vorzüge und Zielsetzungen gemäß der Erfindung offenbart. In den Darstellungen zeigen:
- Fig. 1a: ein Infusions-Set bzw. eine Injektionsvorrichtung mit Merkmalen gemäß der Erfindung in einer schematischen Darstellung in einem axialen Längsschnitt;
- Fig. 1b: die Darstellung gemäß Fig. 1a mit eingezeichnetem Unterdruck- bzw. Vakuumbereich;
- Fig. 2a: einen Kolbenteller mit Merkmalen gemäß der Erfindung in einer axialen Schnittdarstellung;
- Fig. 2b: den Kolbenteller gemäß Fig. 2a in einer perspektivischen Seitenansicht;
- Fig. 3a bis 3e: schematisch in einer Schnittdarstellung die Betätigung einer erfindungsgemäßen Injektionsvorrichtung in verschiedenen Betriebsstadien;
- Fig. 4: einen im Hinblick auf die Erfindung spezialisierten Stopfen in einer axialen Schnittdarstellung;
- Fig. 5a: eine weitere Ausführungsform eines Kolbentellers mit Merkmalen gemäß der Erfindung in einer axialen Schnittdarstellung;
- Fig. 5b: den Kolbenteller gemäß Fig. 5a in einer perspektivischen Seitenansicht; und
- Fig. 6a bis 6f: eine spezielle Ventileinrichtung in einem Kolbenteller gemäß der Erfindung, wobei die Fig. 6a, 6c, 6e eine axiale Schnittdarstellung sind und die Fig. 6b, 6d, 6f Draufsichten, in schematisierter Form wiedergeben.

Nachfolgend werden gleiche oder wenigstens funktionsgleiche Bestandteile mit gleichen oder wenigstens vergleichbaren Bezugszeichen benannt.

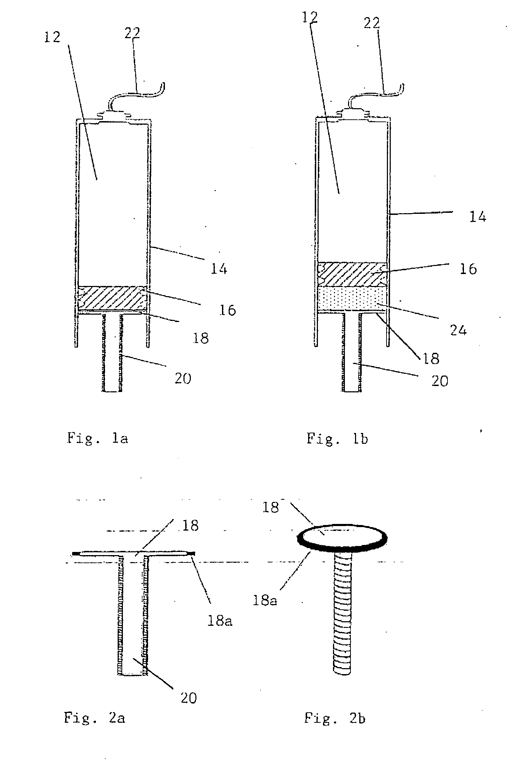

Gemäß Fig. 1a ist innerhalb eines Volumens 14 ein Produkt oder Medikament 12 untergebracht, das über eine Zuführung 22 bzw, ein Infusions-Set 22 das Medikament 12 einem Patienten zuzuführen vermag. Am unteren Ende des Volumens 14, hier eine zylinderförmige Ampulle, ist in deren Inneren ein Stopfen 16 untergebracht, der flüssigkeits- und luftdicht gegenüber der Wandung des Volumens 14 abschließt. An den Stopfen 16 schließt ein Kolbenteller 18 an, an dem eine Kolbenstange 20 zentrisch angebracht ist, Die Gesamtkonfiguration ist zylindersymmetrisch aufgebaut, kann jedoch auch eine andere Form aufweisen. Gemäß Fig. 1a ist der Kolbenteller 18 in unmittelbarer Anlage zu dem Stopfen 16 und es ist noch nicht erkennbar, dass die beiden Bestandteile separat voneinander sind, d.h., nicht miteinander verbunden sind.

Gemäß Fig. 1b ist erkennbar, dass auf die Kolbenstange 20 eine Kraft ausgeübt worden ist. Somit ist der Kolbenteller 18 von dem Stopfen 16 abgezogen worden und es hat sich ein Unterdruck- bzw. Vakuumbereich 24 zwischen dem Kolbenteller 18 und dem Stopfen 16 ausgebildet, der aus illustratorischen Gründen größer dargestellt ist, als er tatsächlich ausfallen würde.

Die Fig. 2a zeigt einen spezifisch ausgebildeten Kolbenteller bzw. Teller 18, an dem wiederum ein Stopfen 16 angebracht ist. Vollumfänglich ist an dem Kolbenteller eine Lippe oder Dichtlippe 18a angebracht. Diese Dichtlippe 18a kann ihre Dichtwirkung zwischen dem Teller 18 und der Wandung des Volumens 14 entfalten. Aus Fig. 2b ist dabei die vollumfängliche Anordnung der Dichtlippe 18a nochmals besser zu ersehen. Die Kolbenstange kann mit einem Gewinde ausgebildet sein, so dass durch eine Drehung der als Gewindestange ausgebildeten Kolbenstange 20 ein Vortrieb für den Kolbenteller 18 bereitgestellt werden kann, bzw. durch eine entgegengesetzte Rotation der Kolbenteller 18 zurückgezogen werden kann. Dabei kann die vollumfänglich vorgesehene Dichtlippe 18a jedenfalls ihre Dichtfunktion entfalten.

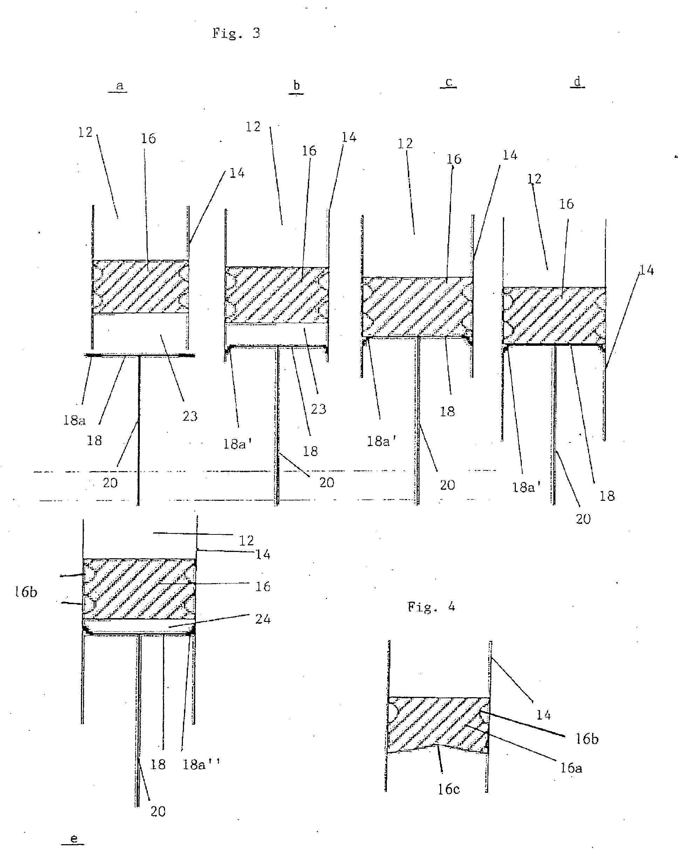

In der Fig. 3 ist das Einführen eines Kolbentellers gemäß der Erfindung in eine Injektionsvorrichtung gemäß der Erfindung in verschiedenen Betriebsstadien wiedergegeben.

Gemäß Fig. 3a ist der Kolbenteller 20 auch außerhalb des Volumens 14 und somit ist die Dichtlippe 18a noch nicht in Anlage zu der Innenoberfläche der Wandung des Volumens 14. Ein Bereich 23 deutet an, dass hier innerhalb des Volumens 14 noch ein Luftvolumen 14 eingerichtet ist. Gemäß Fig. 3b wird nun der Kolbenteller 18 in das Volumen 14 eingeführt, wobei sich die Dichtlippe 18a' nunmehr entgegen der Vorschubrichtung der Kolbenstange 20 verformt. In diesem Verformungszustand ist die Dichtlippe 18a' nur gegen austretendes Gas undicht, so dass die Luft aus dem Volumen 23 entweichen kann. Schließlich kommt gemäß Fig. 3c der Kolbenteller in Kontakt zu dem Stopfen 16 und der Vortrieb für den Kolbenteller 18 endet in dieser Position. Die in ihre Offenstellung verformte Dichtlippe 18a' hat nun nahezu sämtliche vorhandene Luft aus dem nun nicht mehr vorhandenen Bereich 23 ausgelassen. Gegen in den Vakuumsbereich eintretende Luft ist die Dichtlippe in diesem Zustand der Verformung dicht.

Nach der Ausschüttung und zur Entnahme des Volumens wird gemäß Fig. 3d an der Kolbenstange 20 angezogen und zwar entgegen der Einschubrichtung. Sobald die Zugkraft einen kritischen Wert überschreitet, schnappt die Dichtlippe 18a' aus ihrer entgegen der Einschubrichtung ausgerichteten Stellung gemäß Fig. 3d in eine Stellung gemäß Fig. 3e um.

Die Dichtlippe 18a' richtet nun eine nichtdichtende Oberfläche für in den Vakuumsbereich einströmende Luft und ermöglicht somit eine Lösung des Kolbentellers vom Stopfen.

Die Fig. 4 zeigt eine für den gemäß der Erfindung gewünschten Effekt optimierte Form. Die Geometrie des Stopfens 16a ist derart gestaltet, dass das Hereingleiten des Stopfens in das Volumen erheblich kleinere Kräfte erzeugt, als das Herausziehen. Dies ermöglicht ein sicheres und einfacheres Entfernen des Kolbentellers. Der Teller kommt in Fig. 4 von unten in eine teilweise Anlage zu dem Stopfen 16a, während oberhalb des Stopfens das zu verabreichende Produkt gespeichert wird.

Der Stopfen 16 bzw. 16a kann eine oder mehrere radiale Einschnürungen 16b aufweisen, um die Reibung zwischen der Wand des Volumens 14 und dem Stopfen 16, 16a zu verringern. Auch kann eine zusätzliche Dichtwirkung über Ränder zwischen den Einschnürungen 16b und der Wand des Volumens 14 aufgebracht werden. Eine Kavität 16a, die im Stopfen gegenüber dem in Figur 4 nicht dargestellten Kolbenteller 18 dargestellt ist kann vorgesehen sein, um beim Aufbau des Unterdruckbereiches 24 unterstützend zu wirken.

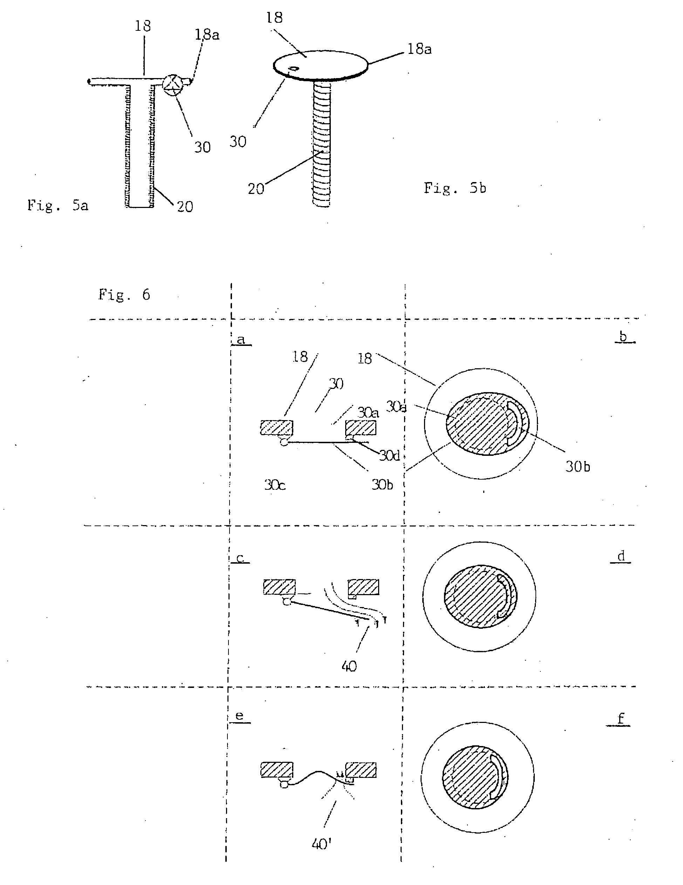

Die Fig. 5a zeigt eine nochmals variierte Form des Kolbentellers 18 nebst Kolbenstange 18. Hier ist eine Ventilvorrichtung 30 in den Teller eingebaut. Eine Dichtwirkung wird von einer angespritzten oder separat vorgesehenen O-Ringdichtung 18a bereitgestellt. Die Position des Ventils 30 geht nochmals aus Fig. 5b hervor.

Natürlich ist es auch möglich, die Kolbenstange 20 hohl auszubilden und an deren oberen Ende einfach mittels eines Stopfens oder eines Verschlusses bzw. einer sonstigen Dichteinrichtung ein Entweichen der Luft aus dem Vakuum- bzw. Unterdruckbereich 24 zu ermöglichen, um dann den Leitungspfad durch die Stange 20 zu verschließen, um das gewünschte Vakuum bzw. den gewünschten Unterdruckbereich 24 bereitzustellen.

In Fig. 6 ist eine spezifisch für Zwecke gemäß der Erfindung ausgebildete Ventileinrichtung dargestellt, die besonders kostengünstig herstellbar ist und dennoch die gewünschte Funktion erfüllen kann. Gemäß Fig. 6a ist die umgebende Tellerwandung des Tellers 18 erkennbar. Ein Loch ist in dem Teller vorgesehen, das hier eine Ventilöffnung darstellt. Das Ventil 30, das dem Ventil gemäß Fig. 5a entsprechen kann, weist eine Ventilöffnung 30a auf. Die Ventilöffnung 30a wird mittels einer Membran 30b abgedichtet, wobei die Membran auf der linken Seite am Ventilteller beispielsweise festgeklebt oder über eine sonstige Festlegungseinrichtung 30c festgelegt ist. Auf der gegenüberliegenden Seite ist die Ventilmembran 30b in Anlage zu einer Anlagedichtung 30d, die beispielsweise aus Silikon, Gummi oder aus Kunststoff ausgebildet sein kann. In einem Ausgangszustand liegt gemäß den Fig. 6a und 6b die Ventilfolie bzw. Ventilmembran 30b, gehalten durch eine Festlegungseinrichtung 30c, an der Anlagedichtung 30d an und wird in Ruhestellung die Öffnung 30a verschließen.

Wird nun der Ventilteller in das Volumen 14 eingeführt, wird gemäß Fig. 6c durch den Überdruck innerhalb des Luft- bzw. Gasvolumens 23 die Ventilmembran 30b ausgelenkt und die Luft kann aus dem Hohlraum zwischen dem Stopfen 16 und dem Teller 18 entweichen. Dabei hebt sich, wie gemäß den Fig. 6c und 6d erkennbar ist, die vorher in Anlage zu der Anlagedichtung 30d befindliche Ventilmembran 30b, z. B. per Vorspannung der Ventilmembran 30b von der Anlagedichtung 30d ab. Die Auslassverformung 40 für die Ventilmembran 30b ermöglicht somit die gewünschte Funktion, die ein Entweichen eines Überdrucks zwischen dem Teller 18 und dem Stopfen 16 ermöglichen soll.

Nachdem die Luft entwichen ist, und sobald an dem Ventilteller 18 angezogen wird, wirkt von außen ein Überdruck gemäß Fig. 6e sowie 6f auf die Ventilmembran 30b, was zu einer Abdicht- bzw. Verschlussverformung 40' für die Ventilmembran 30b führt. Nunmehr kann der gewünschte Unterdruckbereich bzw. Vakuumbereich 24 zwischen dem Teller 18 und dem Stopfen 16 bereitgestellt werden.

### Bezugszeichenliste

- 12: Produkt, Medikament
- 14: Volumen
- 16, 16a: Stopfen
- 16b: Einschnürung
- 16c: Kavität
- 18.: Kolbenteller, Teller
- 18a: Lippe, Dichtlippe
- 18a': Lippe, Dichtlippe mit Auslassverformung
- 18a": Lippe, Dichtlippe mit Dichtverformung
- 20: Kolbenstange
- 22: Zuführung, Infusions-Set
- 23: Luft-, Gasvolumen
- 24: Unterdruck-, Vakuumbereich
- 30: Ventil
- 30a: Ventilöffnung
- 30b: Ventilmembran
- 30c: Festlegungseinrichtung
- 30d: Anlagedichtung
- 40: Auslassverformung
- 40': Dichtverformung für Ventilmembran

## Patentansprüche

1. Injektionsvorrichtung mit einem Volumen (12, 14) zur Aufnahme eines zu verabreichenden Produkts (12), mit einem Kolben (20), um auf das Produkt in dem Volumen einzuwirken, und mit einem Stopfen (16), der zwischen dem produktseitigen Ende des Kolbens und dem im Volumen befindlichen Produkt (12) anordenbar ist, **dadurch gekennzeichnet, dass** der Kolben mit einem gasdicht abdichtbaren Kolbenteller (18) ausgebildet ist, der separat zu dem Stopfen (16) ausgebildet ist, so dass ein Unterdruck- oder Vakuumbereich (24) zwischen dem Kolbenteller und dem Stopfen ausbildbar ist.

2. Injektionsvorrichtung nach Anspruch 1, **dadurch gekennzeichnet, dass** der Kolbenteller (18) eine vollumfängliche Dichtlippe (18a) umfasst, die gegenüber der Volumenwandung im Wesentlichen gasdicht abschließt.

3. Injektionsvorrichtung nach Anspruch 2, **dadurch gekennzeichnet, dass** die Dichtlippe in Einführrichtung des Kolbentellers (18) eine Sollundichtigkeitseinrichtung umfasst.

4. Injektionsvorrichtung nach einem der Ansprüche 1 oder 2, **dadurch gekennzeichnet, dass** die Kolbenstange eine öffen- und/oder verschließbare Hohlleitung aufweist, beispielsweise mit einem Stopfen, einem Ventil (30) oder dgl. verschließbar ist.

5. Injektionsvorrichtung nach einem der Ansprüche 2 bis 4, **dadurch gekennzeichnet, dass** die Dichtlippe stülpbar oder biegbar ausgebildet ist.

6. Injektionsvorrichtung nach einem der Ansprüche 2 bis 5, **dadurch gekennzeichnet, dass** die Dichtlippe derart stülpbar oder biegbar ausgebildet ist, dass beim Einführen der Kolbenstange in das Volumen Luft oder Gas zwischen dem Stopfen (16) und dem Kolbenteller (18) entweichen kann.

7. Injektionsvorrichtung nach einem der Ansprüche 1, 2 oder 4, **dadurch gekennzeichnet, dass** der Teller mit einer Dichteinrichtung, etwa einem O-Ring, ausgestattet ist.

8. Injektionsvorrichtung nach einem der Ansprüche 1 bis 7, **dadurch gekennzeichnet, dass** der Teller (18) und/oder die Kolbenstange (20) mit wenigstens einem Ventil (30) ausgebildet ist.

9. Injektionsvorrichtung nach Anspruch 8, **dadurch gekennzeichnet, dass** das Ventil ein Ein-Wege-Ventil ist.

10. Injektionsvorrichtung nach Anspruch 8, **dadurch gekennzeichnet, dass** das Ventil ein Zwei-Wege-Ventil ist, wobei insbesondere unterschiedliche Auslösedrücke vorgesehen sind, insbesondere mit einem Auslösedruck beim Einschieben des Kolbentellers (18), der niedriger als der Auslösedruck beim Herausziehen des Kolbentellers (18) ist.

11. Injektionsvorrichtung nach Anspruch 8, **dadurch gekennzeichnet, dass** zwei Ventile vorgesehen sind, wobei das eine Ventil einen niedrigeren Auslösedruck hat als das andere Ventil, und wobei eines der Ventile in die eine Richtung und das andere in die entgegengesetzte Richtung wirksam ist.

12. Injektionsvorrichtung nach einem der Ansprüche 8 bis 11, **dadurch gekennzeichnet, dass** die wenigstens eine Ventileinrichtung eine Membran (30b), umfasst, die in Luft- oder Gasauslassrichtung frei oder eingeschränkt frei bewegbar ist, während die Membran in Lufteinlassrichtung abgestützt ist, und in eine dichtende Anlage zu einer Membranabstützung (30d) gelangt, so dass zwischen dem Stopfen (16) und dem Kolbenteller (18) ein Unterdruck- bzw. Vakuumbereich (24) ausbildbar ist.

13. Injektionsvorrichtung nach einem der voranstehenden Ansprüche, **dadurch gekennzeichnet, dass** der Stopfen (16a) wenigstens eine radiale Einschnürung (16b) aufweist.

14. Injektionsvorrichtung nach Anspruch 13, **dadurch gekennzeichnet, dass** die dem Kolbenteller (18) zugewandte Seite des Stopfens (16a) wenigstens eine in Axialrichtung des Stopfens ausgebildete Kavität (16c) umfasst.

## Claims

1. An injection device, comprising: a volume (12, 14) for accommodating a product (12) to be administered; a piston (20) for acting on the product in the volume; and a stopper (16) which can be arranged between the product-end of the piston and the product (12) situated in the volume, **characterised in that** the piston is embodied with a piston disc (18) which can be sealed gas-tight and is embodied separately with respect to the stopper (16), such that a negative pressure region or vacuum region (24) can be formed between the piston disc and the stopper.

2. The injection device according to claim 1, **characterised in that** the piston disc (18) includes a fully circumferential sealing lip (18a) which provides a substantially gas-tight seal with respect to the volume wall.

3. The injection device according to claim 2, **characterised in that** the sealing lip includes a nominal permeability means in the insertion direction of the piston disc (18).

4. The injection device according to any one of claims 1 or 2, **characterised in that** the piston rod comprises a hollow conduit which can be opened and/or closed, and can for example be closed by a stopper, a valve (30) or the like.

5. The injection device according to any one of claims 2 to 4, **characterised in that** the sealing lip is embodied such that it can be drawn over or bent.

6. The injection device according to any one of claims 2 to 5, **characterised in that** the sealing lip is embodied such that it can be drawn over or bent in such a way that air or gas between the stopper (16) and the piston disc (18) can escape as the piston rod is inserted into the volume.

7. The injection device according to any one of claims 1, 2 or 4, **characterised in that** the disc is fitted with a sealing means, for example an O-ring.

8. The injection device according to any one of claims 1 to 7, **characterised in that** the disc (18) and/or the piston rod (20) is embodied with at least one valve (30).

9. The injection device according to claim 8, **characterised in that** the valve is a one-way valve.

10. The injection device according to claim 8, **characterised in that** the valve is a two-way valve, wherein different triggering pressures are in particular are provided, in particular with a triggering pressure when the piston disc (18) is inserted which is lower than the triggering pressure when the piston disc (18) is withdrawn.

11. The injection device according to claim 8, **characterised in that** two valves are provided, wherein one valve has a lower triggering pressure than the other valve, and wherein one of the valves acts in one direction and the other valve acts in the opposite direction.

12. The injection device according to any one of claims 8 to 11, **characterised in that** the at least one valve means includes a membrane (30b) which can be freely moved or freely moved within restrictions in the air or gas outlet direction while the membrane (30b) is supported in the air inlet direction, and enters into a sealing abutment with a membrane support (30d), such that a negative pressure region or vacuum region (24) can be formed between the stopper (16) and the piston disc (18).

13. The injection device according to any one of the preceding claims, **characterised in that** the stopper (16) comprises at least one radial constriction (16b).

14. The injection device according to claim 13, **characterised in that** the side of the stopper (16a) which faces the piston disc (18) includes at least one cavity (16c) which is formed in the axial direction of the stopper.

## Revendications

1. Dispositif d'injection comprenant un volume (12, 14) servant à recevoir un produit à administrer (12), un piston (20) pour agir sur le produit dans le volume, et un bouchon (16) qui peut être disposé entre l'extrémité côté produit du piston et le produit (12) se trouvant dans le volume, **caractérisé en ce que** le piston est réalisé avec une tête de piston (18) pouvant être étanchéifiée de manière étanche au gaz, qui est réalisé de manière séparée par rapport au bouchon (16), de telle sorte qu'une zone de faible pression ou une zone de vide (24) peut être réalisée entre la tête de piston et le bouchon.

2. Dispositif d'injection selon la revendication 1, **caractérisé en ce que** la tête de piston (18) comprend une lèvre étanche (18a) sur l'intégralité de sa périphérie, qui isole essentiellement de manière étanche au gaz par rapport aux parois du volume.

3. Dispositif d'injection selon la revendication 2, **caractérisé en ce que** la lèvre étanche comprend dans le sens d'introduction de la tête de piston (18) un système de non-étanchéité de consigne.

4. Dispositif d'injection selon l'une quelconque des revendications 1 ou 2, **caractérisé en ce que** la tige de piston présente un conduit creux pouvant être ouvert ou fermé, la tige de piston pouvant par exemple être fermée par un bouchon, une valve (30) ou similaire.

5. Dispositif d'injection selon l'une quelconque des revendications 2 à 4, **caractérisé en ce que** la lèvre étanche est réalisée de manière à pouvoir être emboutie ou cintrée.

6. Dispositif d'injection selon l'une quelconque des revendications 2 à 5, **caractérisé en ce que** la lèvre étanche est réalisée de manière à pouvoir être emboutie ou cintrée de telle manière que lors de l'introduction de la tige de piston dans le volume, de l'air ou du gaz peut s'échapper entre le bouchon (16) et la tête de piston (18).

7. Dispositif d'injection selon l'une quelconque des revendications 1, 2 ou 4, **caractérisé en ce que** la tête est réalisé avec un dispositif d'étanchéité, par exemple avec un joint torique.

8. Dispositif d'injection selon l'une quelconque des revendications 1 à 7, **caractérisé en ce que** la tête (18) et/ou la tige de piston (20) sont réalisés avec au moins un clapet (30).

9. Dispositif d'injection selon la revendication 8, **caractérisé en ce que** le clapet est unidirectionnel.

10. Dispositif d'injection selon la revendication 8, **caractérisé en ce que** le clapet est un clapet bidirectionnel, en particulier diverses pressions de déclenchement étant prévues, en particulier avec une pression de déclenchement lors de l'insertion par coulissement du disque de piston (18) plus basse que la pression de déclenchement lors du retrait du disque de piston (18).

11. Dispositif d'injection selon la revendication 8, **caractérisé en ce que** deux clapets sont prévus, sachant que l'un des clapets présente une pression de déclenchement plus basse que l'autre clapet, et sachant que l'un des clapets est actif dans une direction et que l'autre clapet est actif dans la direction opposée.

12. Dispositif d'injection selon l'une quelconque des revendications 8 à 11, **caractérisé en ce que** le dispositif composé de au moins un clapet comprend une membrane (30b) qui peut être déplacée dans la direction d'évacuation de l'air ou du gaz librement ou librement de manière restreinte, tandis que la membrane est supportée dans la direction d'entrée de l'air et accède à un dispositif de support de membrane (30d) dans une installation d'étanchéification, de telle sorte qu'une zone de faible pression ou de vide (24) peut être réalisée entre le bouchon (16) et la tête de piston (18).

13. Dispositif d'injection selon l'une quelconque des revendications précédentes, **caractérisé en ce que** le bouchon (16a) présente au moins un rétrécissement (16b) radial.

14. Dispositif d'injection selon la revendication 13, **caractérisé en ce que** le côté du bouchon (16a) tourné vers la tête de piston (18) comprend au moins une cavité (16c) réalisée dans une direction axiale du bouchon.
